# EUROPÄISCHE PATENTSCHRIFT

(11) **EP 3 797 803 B1**
(45) Veröffentlichungstag und Bekanntmachung des Hinweises auf die Patenterteilung: **14.12.2022**
(21) Anmeldenummer: 20197633.9
(22) Anmeldetag: 23.09.2020
(51) Int. Cl.: A61M 1/16

(54) **BLUTBEHANDLUNGSVORRICHTUNG MIT VERBESSERTER BEUTELGEWICHTÜBERWACHUNG**
BLOOD PROCESSING DEVICE WITH IMPROVED BAG WEIGHT MONITORING
DISPOSITIF DE TRAITEMENT DU SANG AVEC SURVEILLANCE AMÉLIORÉE DU POIDS DES SACS

(30) Priorität: 26.09.2019 DE 102019126047
(43) Veröffentlichungstag der Anmeldung: 31.03.2021
(73) Patentinhaber: B. Braun Avitum AG, 34212 Melsungen (DE)
(72) Erfinder: GOLARITS, István, 1073 Budapest (HU); TÉNYI, Botond, 1037 Budapest (HU)
(74) Vertreter: Winter, Brandl - Partnerschaft mbB

(56) Entgegenhaltungen:
- EP-B1- 1 175 238
- US-B2- 9 999 716

## Beschreibung

Die vorliegende Offenbarung betrifft eine Blutbehandlungsvorrichtung, insbesondere Dialysemaschine, zur Verwendung in (kontinuierlichen) Blutbehandlungs-/ Dialysetherapien, insbesondere Nierenersatztherapien, mit: einem extrakorporalen Blutkreislauf, einem Dialysator und einem Dialysierflüssigkeitskreislauf, wobei der extrakorporale Blutkreislauf und der Dialysierflüssigkeitskreislauf über eine in dem Dialysator vorgesehene Membran, über welche Blut (unter Verwendung einer Dialysierflüssigkeitslösung) gefiltert werden kann, voneinander getrennt sind; zumindest einem Beutel, insbesondere Einwegbeutel, welcher ein Fluid enthält; zumindest einer Wägevorrichtung, insbesondere Wägezelle, welche eingerichtet ist, das Gewicht des zumindest einen Beutels zu messen und zu überwachen; und zumindest einer Fluidpumpe, welche eingerichtet ist, das Fluid aus dem Beutel herauszupumpen oder in den Beutel hineinzupumpen.

### Stand der Technik

Aus dem Stand der Technik sind bereits Blutbehandlungsvorrichtungen bekannt. Zum Beispiel offenbart die EP 0 829 265 B1 eine Blutbehandlungsvorrichtung, welche eine Schnittstelle für ein Einwegschlauchset, eine Vielzahl von Pumpen wie eine Blutpumpe, eine Spritzenpumpe, eine Ultrafiltratpumpe und eine Substitutionspumpe, Wägezellen zum Messen des Gewichts von Beuteln, welche für die Blutbehandlung erforderliche Fluide enthalten, eine Benutzeroberfläche umfassend ein Display mit Touchscreen und eine Steuereinheit zum Steuern der Prozesse der Blutbehandlungsvorrichtung aufweist. Insbesondere offenbart dieses Dokument, dass das Gewicht von Fluid in einem Beutel mit Hilfe von Wägezellen bestimmt wird und mit einem aus der Flussrate berechneten Sollgewicht des Fluids verglichen wird. Wenn sich das tatsächliche Gewicht und das Sollgewicht unterscheiden, wird die Flüssigkeitszufuhr gesteuert, um die Differenz zwischen tatsächlichem Gewicht und Sollgewicht zu verringern. Darüber hinaus wird ein Alarm ausgelöst, wenn das Gewicht des Fluids im Beutel nicht dem erwarteten Gewicht entspricht.

Auch offenbart die WO 1985 001 879 A1 eine Dialysemaschine mit Wägezellen zum Wiegen der Inhalte eines Beutels. Insbesondere soll das Gewicht der Beutel überwacht werden, um ein Systemversagen zu detektieren. Ein Alarm wird in einem Fall erzeugt, in welchem eine Gewichtsabnahme nicht angezeigt wird, obwohl sie vorliegt und somit angezeigt werden sollte, was offenbarungsgemäß bei einem umgeknickten Schlauch der Fall sein kann.

Die US 9 999 716 B2 offenbart Beutel mit Wägevorrichtungen, mittels welchen eine Gewichtszunahme bzw. ein Gewichtsverlust der Beutel gemessen werden können. Die US 9 999 716 B2 lehrt, zunächst geeignete Steuerungsmaßnahmen durchzuführen, wenn eine Gewichtsvariation auftritt.

Die EP 1 175 238 B1 offenbart Behälter mit Wägevorrichtungen. Ein Steuersystem ignoriert ungenaue Messungen eines Fluidgewichts der Behälter, die beispielweise aus unbeabsichtigten Stößen an den Wägevorrichtugen resultieren. Eine signifikante Leckage oder unterbrochene Schlauchverbindungen werden von dem Steuersystem nicht ignoriert.

Weiterer Stand der Technik findet sich in der EP 0 611 228 A2, der US 5,112,298 A, der EP 3 034 112 A1, der WO 2016 104 720 A1 und der US 2011/0160649 A1.

Während einer Blutbehandlung/ Dialysebehandlung kann es vorkommen, dass ein Beutel, welcher ein Fluid enthält, das beispielsweise dem extrakorporalen Blutkreislauf oder dem Dialysierflüssigkeitskreislauf zuzuführen ist (z.B. eine Substitutionslösung), in unbeabsichtigter Weise auf die Wägezelle gedrückt wird, beispielsweise wenn ein Anwender an dem Beutel zieht oder den Beutel zusammendrückt. Wenn auf den Beutel oder auf die Wägezelle gedrückt wird, tritt in üblicher Weise eine Abweichung/ Schwankung/ Fluktuation/ Variation in dem Gewicht des Beutels auf, welches von der Wägezelle gemessen wird.

Der Stand der Technik hat grundsätzlich den Nachteil, dass Abweichungen/ Schwankungen/ Fluktuationen/ Variationen in einem Gewicht des Beutels, welche durch ein unbeabsichtigtes Drücken auf den Beutel oder auf die Wägezelle hervorgerufen werden, nicht geeignet berücksichtigt werden. Insbesondere sieht der Stand der Technik keine geeigneten Maßnahmen vor, wie mit diesem speziellen Störungsfall in geeigneter Weise umgegangen werden kann, insbesondere, wenn er während einer laufenden Blutbehandlungstherapie auftritt.

### Kurzbeschreibung der Offenbarung

Es ist also Aufgabe der vorliegenden Offenbarung, diese Nachteile aus dem Stand der Technik zu vermeiden oder wenigstens zu mildern. Insbesondere soll die Blutbehandlungsvorrichtung so eingerichtet sein, dass Abweichungen/ Schwankungen/ Fluktuationen/ Variationen in einem Gewicht des Beutels, welche durch ein unbeabsichtigtes Drücken auf den Beutel/ auf die Wägezelle/ des Beutels auf die Wägezelle hervorgerufen werden, während einer laufenden Blutbehandlungstherapie geeignet berücksichtigt werden.

Diese Aufgabe wird durch eine Blutbehandlungsvorrichtung gemäß Anspruch 1 gelöst.

Die Blutbehandlungsvorrichtung weist eine Steuereinheit auf, welche eingerichtet ist: eine Unterbrechung einer Fluidversorgung durch Stoppen der Fluidpumpe zu bewirken, wenn eine unerwartete, durch eine Störung, insbesondere ein unbeabsichtigtes Drücken auf den Beutel oder die Wägevorrichtung/ die Wägezelle, hervorgerufene und durch die Wägevorrichtung gemessene Gewichtsvariation /-abweichung /-schwankung /-fluktuation des Beutels auftritt/ festgestellt wird; die Unterbrechung der Fluidversorgung temporär aufrechtzuerhalten, und zwar so lange, bis die Störung verschwindet, um eine Berechnung einer falschen Fluidbilanz /Flüssigkeitsbilanz/ eines falschen Flüssigkeitshaushalts zu vermeiden/ zu verhindern; die Fluidversorgung durch die Fluidpumpe automatisch wieder bzw. neu zu starten, falls sich der Beutel bzw. das Gewicht des Beutels innerhalb einer vorbestimmten Zeit stabilisiert und die Gewichtsvariation einen vorbestimmten ersten Grenzwert nicht überschreitet; und einen Alarm nur zu erzeugen, falls sich der Beutel/ das Gewicht des Beutels innerhalb der vorbestimmten Zeit nicht stabilisiert und/ oder die

Gewichtsvariation/ -abweichung/ -schwankung/ -fluktuation des Beutels den vorbestimmten ersten Grenzwert überschreitet.

Kern der Offenbarung ist es, dass in einem Fall, in welchem eine durch ein unbeabsichtigtes Drücken des Beutels auf die Wägezelle hervorgerufene Störung auftritt, die Fluidversorgung unterbrochen wird, indem die Fluidpumpe von der Steuereinheit angesteuert und gestoppt wird. Nach der Unterbrechung der Fluidversorgung wird überprüft, ob weiterhin eine Variation/ Abweichung/ Schwankung/ Fluktuation in dem Gewicht des Beutels vorliegt. Beispielsweise wird, wenn nur kurzzeitig und versehentlich an dem Beutel gezogen wird bzw. auf ihn gedrückt wird, das von der Wägezelle überwachte Gewicht nur kurz schwanken bzw. fluktuieren und somit von dem tatsächlichen Gewicht des Beutels abweichen und sich bald wieder auf das tatsächliche Beutelgewicht einpendeln. Die Steuereinheit hält die Unterbrechung der Fluidversorgung so lange aufrecht. Wenn sich das Gewicht des Beutels innerhalb einer vorbestimmten Zeit stabilisiert, das heißt, wenn die Variation/ Schwankung/ Abweichung/ Fluktuation in dem Gewicht des Beutels innerhalb der vorbestimmten Zeit verschwindet und wenn gleichzeitig die Gewichtsvariation einen ersten vorbestimmten Grenzwert nicht überschreitet/ überschritten hat, wird die Fluidversorgung wieder gestartet, indem von der Steuereinheit die Fluidpumpe angesteuert und angetrieben wird. Wenn sich das Gewicht des Beutels jedoch innerhalb der vorbestimmten Zeit nicht stabilisiert, das heißt, wenn die Variation/ Schwankung/ Abweichung/ Fluktuation in dem Gewicht des Beutels innerhalb der vorbestimmten Zeit nicht verschwindet, und/oder wenn die Gewichtsvariation den vorbestimmten ersten Grenzwert überschreitet, erzeugt die Steuereinheit einen Alarm und die Blutbehandlungstherapie wird vorzugsweise abgebrochen.

In anderen Worten wird gemäß der vorliegenden Offenbarung der Alarm ausgegeben und die Blutbehandlungstherapie bevorzugt abgebrochen, wenn die Gewichtsvariation (zu einem beliebigen Zeitpunkt) den ersten vorbestimmten Grenzwert, welcher beispielsweise auf 40 Gramm eingestellt werden kann, überschreitet, das heißt wenn die Gewichtsvariation übermäßig groß ist. Weiterhin wird offenbarungsgemäß der Alarm ausgegeben und die Blutbehandlungstherapie bevorzugt abgebrochen (das heißt auch, dass die Fluidversorgung unterbrochen bleibt), wenn sich das Gewicht (die Masse) des Beutels nicht innerhalb der vorbestimmten Zeit, welche beispielsweise auf eine Minute eingestellt werden kann, stabilisiert. Es versteht sich von selbst, dass der Alarm auch ausgegeben wird und die Blutbehandlungstherapie bevorzugt abgebrochen wird, wenn sowohl die Gewichtsvariation den ersten vorbestimmten Grenzwert überschreitet, als auch sich das Gewicht des Beutels nicht innerhalb der vorbestimmten Zeit stabilisiert. Lediglich wenn sowohl der erste vorbestimmte Grenzwert nicht überschritten wird als auch sich das Gewicht des Beutels innerhalb der vorbestimmten Zeit stabilisiert, wird die Fluidversorgung durch die Fluidpumpe automatisch wiederaufgenommen und die Blutbehandlungstherapie wird fortgesetzt.

Die vorliegende Offenbarung stellt somit eine Blutbehandlungsvorrichtung bereit, welche Abweichungen/ Schwankungen/ Fluktuationen/ Variationen in einem Gewicht des Beutels, welche durch ein unbeabsichtigtes Drücken auf den Beutel oder auf die Wägezelle hervorgerufen werden, während einer laufenden Blutbehandlungstherapie geeignet berücksichtigt.

Vorteilhafte Ausführungsformen sind in den Unteransprüchen beansprucht und werden nachfolgend erläutert.

Es ist vorteilhaft, wenn die Steuereinheit eingerichtet ist, eine unerwartete Gewichtsvariation in dem Beutel festzustellen, wenn von der Wägezelle eine abrupte/ plötzliche Gewichtserhöhung oder Gewichtsverringerung festgestellt wird (eine abrupte Erhöhung oder Verringerung in einem ungefilterten Gewichtswert des Beutels), wobei die Gewichtserhöhung oder die Gewichtsverringerung größer oder gleich einem vorbestimmten Wert ist. Insbesondere konnte gemäß der vorliegenden Offenbarung durch Versuche ein vorbestimmter Wert gefunden werden, welcher in geeigneter Weise Störungen erfasst, die durch ein unbeabsichtigtes Drücken des Beutels auf die Wägezelle hervorgerufen werden. Der vorbestimmte Wert ist dabei bevorzugt zwischen 20 Gramm und 35 Gramm eingestellt. Es hat sich durch Versuche herausgestellt, dass ein Ziehen oder Drücken an dem Beutel meist eine Variation/ Abweichung/ Schwankung/ Fluktuation von über 35 Gramm hervorruft. Somit wird die Fluidversorgung bei kleineren Schwankungen/ Abweichungen nicht unterbrochen, sondern erst, wenn eine Schwankung/ Abweichung vorliegt, bei welcher darauf geschlossen werden kann, dass diese geeignet ist, die von der Steuereinheit durchgeführte Berechnung der Fluidbilanz/ Flüssigkeitsbilanz bzw. des Flüssigkeitshaushalts zu verfälschen.

In vorteilhafter Weise ist die Steuereinheit eingerichtet, die Fluidversorgung durch Stoppen der Fluidpumpe zu unterbrechen, wenn sich der Beutel bzw. das Gewicht des Beutels nicht innerhalb von einer vorbestimmten Zeitspanne, insbesondere etwa 10 Sekunden, stabilisiert. Das heißt, es kann offenbarungsgemäß vorgesehen sein, dass die Unterbrechung der Fluidversorgung (durch ein Stoppen der Fluidpumpe) nicht unmittelbar/ sofort bewirkt wird, wenn die Gewichtsvariation des Beutels festgestellt wird. Bevorzugt wird gemäß der vorliegenden Offenbarung noch kurz gewartet (beispielsweise 10 Sekunden). Wenn sich das Gewicht des Beutels bereits innerhalb dieser kurzen Zeitspanne wieder stabilisiert, ist es nicht erforderlich, die Fluidversorgung zu unterbrechen. Wenn sich das Gewicht des Beutels innerhalb dieser kurzen Zeitspanne nicht wieder stabilisiert, wird bevorzugt die Fluidversorgung durch die Fluidpumpe gestoppt. Die vorliegende Offenbarung ist jedoch nicht auf diese Ausführungsform beschränkt und es kann grundsätzlich auch vorgesehen sein, dass die Unterbrechung der Fluidversorgung (durch ein Stoppen der Fluidpumpe) unmittelbar/ sofort bewirkt wird.

Es ist von Vorteil, wenn die Steuereinheit den Alarm erzeugt, wenn sich der Beutel/ das Gewicht des Beutels nicht innerhalb einer vorbestimmten Zeit, insbesondere innerhalb von einer Minute, stabilisiert. Es hat sich herausgestellt, dass sich das Gewicht des Beutels innerhalb eines relativ kurzen Zeitraums wieder stabilisiert, wenn die Variation/ Schwankung/ Abweichung/ Fluktuation in dem Gewicht des Beutels dadurch hervorgerufen wird, dass in unbeabsichtigter Weise kurz auf den Beutel/ auf die Wägezelle gedrückt wird. Liegt die Variation/ Schwankung/ Abweichung/ Fluktuation länger vor, kann es sein, dass der Beutel z.B. dauerhaft eingeklemmt wurde, so dass die Blutbehandlungstherapie zu unterbrechen ist und der betroffene Beutel von einem Anwender untersucht werden kann.

Ein besonders bevorzugtes Ausführungsbeispiel der vorliegenden Erfindung ist dadurch gekennzeichnet, dass die Steuereinheit eingerichtet ist, eine unerwartete Gewichtsvariation in dem Beutel festzustellen, wenn von der Wägezelle eine abrupte/ plötzliche Gewichtserhöhung oder Gewichtsverringerung um mehr als 35 Gramm festgestellt wird, und den Alarm zu erzeugen, wenn sich der Beutel/ das Gewicht des Beutels nicht innerhalb von einer Minute stabilisiert.

Es ist von Vorteil, wenn die Steuereinheit eingerichtet ist, eine durch ein unbeabsichtigtes Drücken auf die Wägezelle oder den Beutel hervorgerufene Gewichtsvariation festzustellen.

In vorteilhafter Weise ist die Steuereinheit eingerichtet, wenn die unerwartete Gewichtsvariation des Beutels festgestellt bzw. erfasst wird, die Berechnung der Fluidbilanz sofort bzw. unmittelbar zu stoppen. Somit wird die Variation in dem Gewicht des Beutels in geeigneter Weise berücksichtigt und nicht fälschlicherweise in die Fluidbilanz mit einberechnet. Es ist grundsätzlich wünschenswert, dass die Fluidbilanz korrekt berechnet wird, damit das Verhältnis zwischen entzogener Menge an Flüssigkeit und zurückgegebener Menge an Flüssigkeit gemäß den medizinischen Erfordernissen geeignet eingestellt werden kann.

Bevorzugt ist die Steuereinheit somit eingerichtet, wenn die unerwartete Gewichtsvariation des Beutels festgestellt bzw. erfasst wird, die Berechnung der Fluidbilanz sofort bzw. unmittelbar zu stoppen, und die Fluidversorgung durch Stoppen der Fluidpumpe erst zu unterbrechen, wenn sich der Beutel bzw. das Gewicht des Beutels nicht innerhalb von einer vorbestimmten Zeitspanne, insbesondere etwa 10 Sekunden, stabilisiert. In anderen Worten wird somit bei einem Erfassen einer unerwarteten Gewichtsvariation die Berechnung der Fluidbilanz sofort gestoppt, während hingegen die Fluidversorgung durch die Fluidpumpe nicht sofort gestoppt wird, sondern noch eine kurze Zeitspanne gewartet wird. Wenn sich die Gewichtsvariation innerhalb dieser kurzen Zeitspanne wieder stabilisiert, und somit kein Stoppen der Fluidversorgung erforderlich wird, wird die Gewichtsvariation einfach im Nachhinein in die Fluidbilanz einberechnet.

Es ist von Vorteil, wenn (in dem Fall, dass die Fluidversorgung durch Stoppen der Fluidpumpe unterbrochen ist) die Steuereinheit eingerichtet ist, die Gewichtsvariation in die Fluidbilanz einzuberechnen, falls sich der Beutel bzw. das Gewicht des Beutels innerhalb der vorbestimmten Zeit stabilisiert und die Gewichtsvariation den ersten vorbestimmten Grenzwert nicht überschreitet.

Offenbarungsgemäß kann es somit vorgesehen sein, dass die Gewichtsvariation (im Nachhinein) in die Fluidbilanz mit einberechnet wird, und zwar insbesondere, wenn die Fluidversorgung durch die Fluidpumpe fortgesetzt wird, das heißt überhaupt nicht unterbrochen wird, oder wenn die Fluidversorgung durch die Fluidpumpe nach einer Unterbrechung wiederaufgenommen wird.

In vorteilhafter Weise ist die Steuereinheit somit eingerichtet, falls die Gewichtsvariation den vorbestimmten ersten Grenzwert nicht überschreitet, das heißt kleiner oder gleich dem ersten vorbestimmten Grenzwert ist, und sich der Beutel bzw. das Gewicht des Beutels innerhalb der vorbestimmten Zeit stabilisiert, die Fluidversorgung durch die Fluidpumpe automatisch wieder bzw. neu zu starten, keinen Alarm auszugeben und die Gewichtsvariation in die Fluidbilanz einzuberechnen.

Weiterhin ist die Steuereinheit bevorzugt eingerichtet, falls die Gewichtsvariation den vorbestimmten ersten Grenzwert überschreitet und einen vorbestimmten zweiten Grenzwert nicht überschreitet, das heißt zwischen dem ersten vorbestimmten Grenzwert und dem zweiten vorbestimmten Grenzwert liegt, und sich der Beutel bzw. das Gewicht des Beutels innerhalb der vorbestimmten Zeit stabilisiert, den Alarm zu erzeugen und einen Anwender über eine Benutzerschnittstelle zu fragen, ob er die Gewichtsvariation in die Fluidbilanz einberechnen möchte oder nicht. In diesem speziellen Fall, in welchem sich das Gewicht des Beutels grundsätzlich wieder stabilisiert, und auch die Gewichtsvariation noch nicht zu groß war (kleiner dem vorbestimmten zweiten Grenzwert), kann der Anwender somit in vorteilhafter Weise im Nachhinein durch eine entsprechende Eingabe entscheiden, ob er die Gewichtsvariation in die Fluidbilanz einberechnen möchte. Dies geht in der Praxis regelmäßig einher mit einer Wiederaufnahme der Blutbehandlungstherapie durch den Anwender. Alternativ kann der Anwender jedoch auch entscheiden, dass er die Gewichtsvariation nicht in die Fluidbilanz einberechnen möchte. Dies geht in der Praxis regelmäßig einher mit einem Abbruch der Blutbehandlungstherapie.

Bevorzugt ist die Steuereinheit eingerichtet, falls die Gewichtsvariation den vorbestimmten ersten Grenzwert überschreitet und einen/ den vorbestimmen zweiten Grenzwert nicht überschreitet, das heißt zwischen dem ersten vorbestimmten Grenzwert und dem zweiten vorbestimmten Grenzwert liegt, und sich der Beutel bzw. das Gewicht des Beutels innerhalb der vorbestimmten Zeit nicht stabilisiert, den Alarm zu erzeugen, die Fluidversorgung unterbrochen zu halten und die Gewichtsvariation nicht in die Fluidbilanz einzuberechnen.

Die Steuereinheit ist ferner bevorzugt eingerichtet, falls die (unerwartete ungefilterte) Gewichtsvariation des Beutels einen/ den zweiten vorbestimmten Grenzwert überschreitet, das heißt größer dem zweiten vorbestimmten Grenzwert ist, einen sofortigen Alarm zu erzeugen, die Fluidversorgung zu unterbrechen oder unterbrochen zu halten und die Gewichtsvariation nicht in die Fluidbilanz einzuberechnen.

In vorteilhafter Weise kann die Steuereinheit auch eingerichtet sein, wenn sich das (ungefilterte) Gewicht des Beutels innerhalb der vorbestimmten Zeit stabilisiert, jedoch die (unerwartete ungefilterte) Gewichtsvariation des Beutels einen/ den zweiten vorbestimmten Grenzwert überschreitet, den Alarm zu erzeugen und die Gewichtsvariation nicht in die Fluidbilanz einzuberechnen.

Bevorzugt ist die vorbestimmte Zeit (bevorzugt eine Minute) größer als die angesprochene vorbestimmte Zeitspanne (etwa 10 Sekunden). Insbesondere ist die vorbestimmte Zeit eine Minute bzw. 60 Sekunden und ist die vorbestimmte Zeitspanne 10 Sekunden.

Bevorzugt ist der vorbestimmte zweite Grenzwert größer als der vorbestimmte erste Grenzwert, und der vorbestimmte erste Grenzwert ist größer als der vorbestimmte Wert (zwischen 20 und 35 Gramm, insbesondere 35 Gramm). Der vorbestimmte erste Grenzwert ist bevorzugt 40 Gramm. Weiter bevorzugt ist der vorbestimmte zweite Grenzwert bevorzugt 80 Gramm.

Der extrakorporale Blutkreislauf und der Dialysierflüssigkeitskreislauf sind bevorzugt als Einwegschläuche ausgebildet, welche an einer an der Dialysemaschine vorgesehenen Schnittstelle aufgenommen sind.

Es ist zweckmäßig, wenn die Blutbehandlungsvorrichtung ein Bar-Code-Lesegerät aufweist, welches eingerichtet ist, auf Einwegartikel wie etwa Einwegschläuche oder auf deren Umverpackung aufgebrachte Bar-Codes zu lesen.

Darüber hinaus ist es zweckmäßig, wenn die Blutbehandlungsvorrichtung eine Benutzeroberfläche umfassend ein Display mit Touch-Screen hat.

Die Blutbehandlungsvorrichtung ist bevorzugt zur drahtgebundenen Kommunikation eingerichtet ist.

Die Steuereinheit der Blutbehandlungsvorrichtung ist vorzugsweise als zumindest ein Prozessor, vorzugsweise mehrere Prozessoren, ausgebildet.

Mit anderen Worten betrifft die Offenbarung eine Dialysemaschine. Die Dialysemaschine enthält ein Bar-Code-Lesegerät. Weiterhin enthält die Dialysemaschine eine Benutzeroberfläche bzw. ein Display mit Touch-Screen. Die Dialysemaschine weist ferner eine Schnittstelle/ Interface für ein Einwegschlauchset auf, welches eine Blutseite und eine Dialysierflüssigkeitsseite enthält, die voneinander durch eine (semi-) permeable/ durchlässige Membran getrennt sind, um Blut unter Verwendung einer Dialysierflüssigkeitslösung/ Dialyselösung zu filtern. Eine Substitutionslösung/ Ersatzlösung wird der Blutseite vor/nach einem Dialysator zugeführt. Die Dialysemaschine weist eine Blutpumpe, eine Spritzenpumpe, eine Ultrafiltratpumpe, eine Substitutionslösungspumpe etc. auf. Die Dialysemaschine ist zur drahtgebundenen Kommunikation eingerichtet/ weist verdrahtete bzw. drahtgebundene Kommunikationseinrichtungen auf. Die Dialysemaschine zeichnet sich durch eine Software aus, welche besonders für eine Verwendung in kontinuierlichen Dialysetherapien, beispielsweise Nierenersatztherapien geeignet ist. Die Software läuft dabei auf einer Vielzahl von Prozessoren innerhalb der Dialysemaschine. Weiterhin weist die Dialysemaschine eine Energieverwaltungseinrichtung (integrierter Schaltkreis) auf. Die Dialysemaschine enthält darüber hinaus Wägevorrichtungen, insbesondere Wägezellen, welche das Gewicht von Einwegbeuteln messen, welche die für die Dialysetherapie erforderlichen Fluide (z.B. Dialysierflüssigkeitslösung, Substitutionslösung) enthalten.

Die vorliegende Offenbarung stellt insbesondere eine verbesserte Beutelgewichtüberwachung bereit. Wenn Beutel in unbeabsichtigter Weise auf die Wägezelle gedrückt werden oder eine andere (ähnliche) unerwartete Fluktuation erfasst wird, stoppt die Blutbehandlungsvorrichtung (eine Steuereinheit derselben) automatisch die Fluidpumpe/ die Fluidpumpen und unterbricht die Therapie. Um zu verhindern, dass die Maschine/ Dialysemaschine/ Blutbehandlungsvorrichtung (die Steuereinheit derselben) eine falsche Fluidbilanz/ ein falsches Flüssigkeitsgleichgewicht berechnet, wird diese Unterbrechung aufrechterhalten, bis die Störung verschwindet. Falls der Beutel sich nicht spätestens innerhalb von einer Minute stabilisiert/ Falls der Beutel nicht spätestens innerhalb einer Minute stabilisiert wird, wird ein Alarm erzeugt. Gemäß einer bevorzugten Ausführungsform wird ein Alarm erzeugt, wenn ein instabiles Gewicht, welches größer als 35 Gramm ist, vorliegt und die Fluktuation in dem Gewicht länger als eine Minute dauert.

### Kurzbeschreibung der Figuren

Die Offenbarung wird nachfolgend mit Hilfe von Figuren weiter erläutert. Es zeigen:
- Fig. 1: eine schematische Ansicht einer Blutbehandlungsvorrichtung gemäß der vorliegenden Offenbarung;
- Fig. 2: ein Flussdiagramm, welches die offenbarungsgemäße, in der Steuereinheit ablaufende verbesserte Beutelgewichtüberwachung veranschaulicht; und
- Fig. 3: ein Diagramm, in welchem ein zeitlicher Verlauf eines Gewichts eines Beutels und einer Flussrate einer Fluidpumpe, welche ein Fluid aus dem Beutel herauspumpt oder in den Beutel hineinpumpt, gemäß der vorliegenden Offenbarung dargestellt ist.

### Figurenbeschreibung

Die Figuren sind lediglich schematischer Natur und dienen ausschließlich dem Verständnis der vorliegenden Offenbarung. Gleiche Elemente sind dabei mit denselben Bezugszeichen versehen.

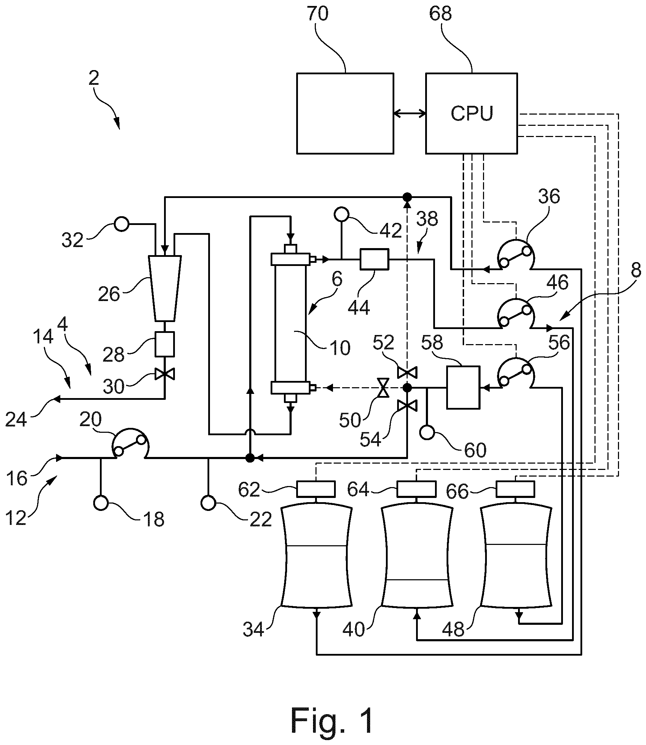

Fig. 1 zeigt eine schematische Ansicht einer extrakorporalen Blutbehandlungsvorrichtung (Dialysemaschine) 2. Die Blutbehandlungsvorrichtung 2 ist grundsätzlich eingerichtet, sowohl in kontinuierlichen als auch in intermittierenden Blutbehandlungstherapien, insbesondere Nierenersatztherapien, verwendet zu werden. Die Blutbehandlungsvorrichtung 2 ist insbesondere als eine Akutdialysemaschine bzw. als ein Akut-Dialysegerät ausgebildet und ist somit im Wesentlichen zur Verwendung auf Intensivstationen bei überwiegend instabilen Patienten vorbereitet. Mit der Blutbehandlungsvorrichtung 2 der vorliegenden Offenbarung können grundsätzlich eine Vielzahl von verschiedenen Blutbehandlungstherapien (z.B. langsame kontinuierliche Ultrafiltration (SCUF), kontinuierliche venös-venöse Hämofiltration (CVVH), kontinuierliche venös-venöse Hämodialyse (CVVHD), kontinuierliche venös-venöse Hämodiafiltration (CVVHDF), therapeutischer Plasmaaustauch (TPE), etc.), Dilutionsmodi (z.B. Prädilution, Postdilution, Prä- und Postdilution), sowie Antikoagulationsarten (z.B. keine, Heparin, Citrat, etc.) durchgeführt werden.

Die Blutbehandlungsvorrichtung 2 weist grundsätzlich einen extrakorporalen Kreislauf 4, einen Dialysator (Hämofilter) 6 und einen Dialysierflüssigkeitskreislauf 8 auf. Der extrakorporale Kreislauf 4 und der Dialysierflüssigkeitskreislauf 8 sind über eine in dem Dialysator 6 vorgesehene Membran 10, über welche Blut unter Verwendung einer Dialysierflüssigkeitslösung oder ohne Verwendung einer Dialysierflüssigkeitslösung gefiltert werden kann, voneinander getrennt.

Der extrakorporale Kreislauf 4 umfasst einen arteriellen Abschnitt 12 und einen venösen Abschnitt 14. Dabei ist grundsätzlich vorgesehen, dass der arterielle Abschnitt 12, insbesondere ein Ende desselben, an eine Arterie eines Patienten, insbesondere eines Intensivpatienten, angeschlossen bzw. mit derselben verbunden werden soll. Weiterhin ist vorgesehen, dass der venöse Abschnitt 14, insbesondere ein Ende desselben, an eine Vene eines Patienten, insbesondere eines Intensivpatienten, angeschlossen bzw. mit derselben verbunden werden soll.

Der arterielle Abschnitt 12 weist ausgehend von einem arteriellen Ende 16 in einer Blut-Strömungsrichtung hin zu dem Dialysator 6 einen arteriellen Drucksensor 18, eine (arterielle) Blutpumpe 20 und einen Dialysatoreingangsdrucksensor 22 auf. Der venöse Abschnitt 14 weist ausgehend von dem Dialysator 6 in einer Blut-Strömungsrichtung hin zu einem venösen Ende 24 eine venöse Expansionskammer bzw. Luftfalle 26, einen Sicherheitsluftdetektor 28 und ein Sicherheitsventil 30 auf. An/ hinter der venösen Expansionskammer 26 kann ein venöser Druck über einen venösen Drucksensor 32 gemessen werden.

Wie aus Fig. 1 hervorgeht, ist die venöse Expansionskammer 26 mit einem Substitutionslösungsbeutel/ -behälter 34 verbunden. Eine Substitutionslösungspumpe 36 ist dabei vorgesehen und eingerichtet, eine Substitutionslösung aus dem Substitutionslösungsbeutel 34 in den extrakorporalen Blutkreislauf 4, insbesondere in den venösen Abschnitt 14 desselben (in die venöse Expansionskammer 26) zu pumpen.

Der Dialysierflüssigkeitskreislauf 8 weist zumindest einen Abfluss 38 für Ultrafiltrat/ verbrauchte Dialysierflüssigkeit (Dialysat)/ eine sonstige Flüssigkeit auf. Grundsätzlich kann das Ultrafiltrat/ Dialysat/ die sonstige Flüssigkeit über den Abfluss 38 von dem Dialysator 6 hin zu einem Sammelbeutel/ -behälter 40 für Ultrafiltrat/ Dialysat/ etc. fließen. In dem Abfluss 38 sind ausgehend von dem Dialysator 6 in einer Strömungsrichtung hin zu dem Sammelbeutel 40 ein Ultrafiltratdrucksensor 42, ein Blutleckdetektor 44 und eine Ultrafiltratpumpe 46 angeordnet bzw. vorgesehen.

Wie aus Fig. 1 weiter hervorgeht, ist neben dem Substitutionslösungsbeutel 34 und dem Sammelbeutel 40 noch ein weiterer Beutel/ Behälter 48 vorgesehen. Der Beutel 48 kann in Abhängigkeit von der gewünschten durchzuführenden Blutbehandlungstherapie beispielsweise eine Substitutionslösung /-flüssigkeit oder eine Dialysierflüssigkeit enthalten.

Wenn beispielsweise mit der extrakorporalen Blutbehandlungsvorrichtung 2 eine Hämodialyse-/ eine Hämodiafiltrationsbehandlung etc. durchgeführt werden soll, also eine Blutbehandlungstherapie, in welcher Dialysierflüssigkeit durch den Dialysator 6 strömt und somit ein Stofftransport von dem extrakorporalen Kreislauf 4 zu dem Dialysierflüssigkeitskreislauf 8 sowohl über Diffusion als auch über Konvektion erfolgt, enthält der Beutel 48 Dialysierflüssigkeit. Wird nun ein erstes Ventil 50 geöffnet und werden sowohl ein zweites Ventil 52 als auch ein drittes Ventil 54 geschlossen, kann die Dialysierflüssigkeit über eine Pumpe 56 zum Dialysator 6 gepumpt werden.

Soll alternativ mit der extrakorporalen Blutbehandlungsvorrichtung 2 beispielsweise eine Hämofiltration etc. durchgeführt werden, also eine Blutbehandlungstherapie, in welcher keine Dialysierflüssigkeit durch den Dialysator 6 strömt und somit ein Stofftransport von dem extrakorporalen Kreislauf 4 zu dem Dialysierflüssigkeitskreislauf 8 lediglich über Konvektion/ Filtration erfolgt, kann der Beutel 48 eine Substitutionslösung enthalten. Werden nun das erste Ventil 50 und das zweite Ventil 52 geschlossen und wird das dritte Ventil 54 geöffnet, kann die Substitutionslösung aus dem Beuel 48 in den arteriellen Abschnitt 12 des extrakorporalen Kreislaufs 4 gepumpt werden (Prädilution). Werden das erste Ventil 50 und das dritte Ventil 54 geschlossen und wird das zweite Ventil 52 geöffnet, kann die Substitutionslösung aus dem Beuel 48 in den venösen Abschnitt 14 des extrakorporalen Kreislaufs 4 gepumpt werden (Postdilution). Wird das erste Ventil 50 geschlossen und werden das zweite Ventil 52 und das dritte Ventil 54 geöffnet, kann die Substitutionslösung aus dem Beutel 48 sowohl in den arteriellen Abschnitt 12 als auch in den venösen Abschnitt 14 des extrakorporalen Kreislaufs gepumpt werden (Prä- und Postdilution). Eine Prä- und Postdilution kann gemäß der vorliegenden Offenbarung auch dadurch erreicht werden, dass die Substitutionslösung aus dem Substitutionslösungsbeutel 34 mittels der Substitutionslösungspumpe 36 in den venösen Abschnitt 14 des extrakorporalen Kreislaufs 4 gepumpt wird (Postdilution), und dass gleichzeitig die Substitutionslösung aus dem Beutel 48 mittels der Pumpe (Substitutionslösungspumpe) 56 in den arteriellen Abschnitt 12 des extrakorporalen Kreislaufs 4 gepumpt wird (Prädilution).

Wie aus Fig. 1 weiter hervorgeht, sind zwischen der Pumpe 56 und der Ventilanordnung bestehend aus dem ersten Ventil 50, dem zweiten Ventil 52 und dem dritten Ventil 54 ein Flüssigkeitswärmer 58 und ein Drucksensor 60 vorgesehen.

An den drei Beuteln, also dem Substitutionslösungsbeutel 34, dem Sammelbeutel 40 und dem Beutel 48, sind jeweils Wägezellen, nämlich eine erste Wägezelle 62, eine zweite Wägezelle 64 und eine dritte Wägezelle 66 angeordnet. Die erste Wägezelle 62 ist dabei grundsätzlich eingerichtet, das Gewicht des Substitutionslösungsbeutels 34 zu messen bzw. zu überwachen. Die zweite Wägezelle 64 ist grundsätzlich eingerichtet, das Gewicht des Sammelbeutels 40 zu messen bzw. zu überwachen. Die dritte Wägezelle 66 ist grundsätzlich eingerichtet, das Gewicht des Beutels 48 zu messen bzw. zu überwachen. Die Wägezellen 62, 64, 66 sind grundsätzlich Beispiele von Wägevorrichtungen. Die vorliegende Offenbarung ist jedoch nicht darauf beschränkt, dass die Wägevorrichtungen als Wägezellen 62, 64, 66 ausgebildet sind. Grundsätzlich kann auch eine beliebige andere Wägevorrichtung/ eine Waage/ ein Kraftaufnehmer vorgesehen sein, solange sie/ er es ermöglicht, das Gewicht/ die Masse eines Beutels zu messen bzw. zu überwachen.

Die extrakorporale Blutbehandlungsvorrichtung 2 weist weiterhin eine Steuereinheit (CPU) 68 auf, welche Informationen von den in der Blutbehandlungsvorrichtung 2 vorgesehenen Sensoren erhält, und welche die in der Blutbehandlungsvorrichtung 2 vorgesehenen Aktoren ansteuert. Offenbarungsgemäß wird somit insbesondere eine softwareunterstützte Therapie bereitgestellt. Die Steuereinheit 68 erhält dabei insbesondere Informationen von dem arteriellen Drucksensor 18, dem Dialysatoreingangsdrucksensor 22, dem Sicherheitsluftdetektor 28, dem venösen Drucksensor 32, dem Ultrafiltratdrucksensor 42, dem Blutleckdetektor 44, dem Drucksensor 60, der ersten Wägezelle 62, der zweiten Wägezelle 64, der dritten Wägezelle 66, etc. Die Steuereinheit 68 steuert insbesondere die Blutpumpe 20, das Sicherheitsventil 30, die Substitutionslösungspumpe 36, die Ultrafiltratpumpe 46, das erste Ventil 50, das zweite Ventil 52, das dritte Ventil 54, die Pumpe 56, den Flüssigkeitswärmer 58, etc. an. Weiterhin ist die Steuereinheit 68 im Austausch mit einer als Display mit Touch-Screen ausgebildeten Benutzeroberfläche 70. Beispielsweise kann die Steuereinheit 68 eingerichtet sein, eine Warnung oder einen Alarm auf der Benutzeroberfläche 70 anzuzeigen. Weiterhin können von einem Nutzer/ Anwender auf der Benutzeroberfläche 70 eingegebene Informationen an die Steuereinheit 68 weitergegeben werden.

Wie aus Fig. 1 bereits hervorgeht, betrifft die vorliegende Offenbarung im Wesentlichen die Ansteuerung der Substitutionslösungspumpe 36, der Ultrafiltratpumpe 46 und der Pumpe 56 auf der Grundlage der von der ersten Wägezelle 62, der zweiten Wägezelle 64 und der dritten Wägezelle 66 erhaltenen Informationen. Die vorliegende Offenbarung betrifft insgesamt im Wesentlichen eine durch die Steuereinheit 68 vorgenommene Steuerung. Wenn von der Steuereinheit 68 auf der Grundlage von von der ersten Wägezelle 62 übermittelten Informationen festgestellt/ gemessen wird, dass eine unerwartete Gewichtsvariation des Substitutionslösungsbeutels 34 auftritt, steuert die Steuereinheit 68 die Substitutionslösungspumpe 36 an, um diese zu stoppen, um so eine Unterbrechung der Fluidversorgung zu bewirken. Wenn von der Steuereinheit 68 auf der Grundlage von von der zweiten Wägezelle 64 übermittelten Informationen festgestellt/ gemessen wird, dass eine unerwartete Gewichtsvariation des Sammelbeutels 40 auftritt, steuert die Steuereinheit 68 die Ultrafiltratpumpe 46 an, um diese zu stoppen, um so eine Unterbrechung der Fluidversorgung zu bewirken. Wenn von der Steuereinheit 68 auf der Grundlage von von der dritten Wägezelle 66 übermittelten Informationen festgestellt/ gemessen wird, dass eine unerwartete Gewichtsvariation des Beutels 48 auftritt, steuert die Steuereinheit 68 die Pumpe 56 an, um diese zu stoppen, um so eine Unterbrechung der Fluidversorgung zu bewirken.

Es gilt somit grundsätzlich, dass, wenn gemäß der vorliegenden Offenbarung allgemein von einem Beutel die Rede ist, sowohl der Substitutionslösungsbeutel 34, als auch der Sammelbeutel 40, als auch der Beutel 48 gemeint sein kann. Weiterhin gilt, dass, wenn gemäß der vorliegenden Offenbarung allgemein von einer Wägevorrichtung oder von einer Wägezelle die Rede, ist, sowohl die erste Wägezelle 62, als auch die zweite Wägezelle 64 als auch die dritte Wägezelle 66 gemeint sein kann. Darüber hinaus gilt, dass wenn gemäß der vorliegenden Offenbarung allgemein von einer Fluidpumpe die Rede ist, sowohl die Substitutionslösungspumpe 36, als auch die Ultrafiltratpumpe 46, als auch die Pumpe 56 gemeint sein kann.

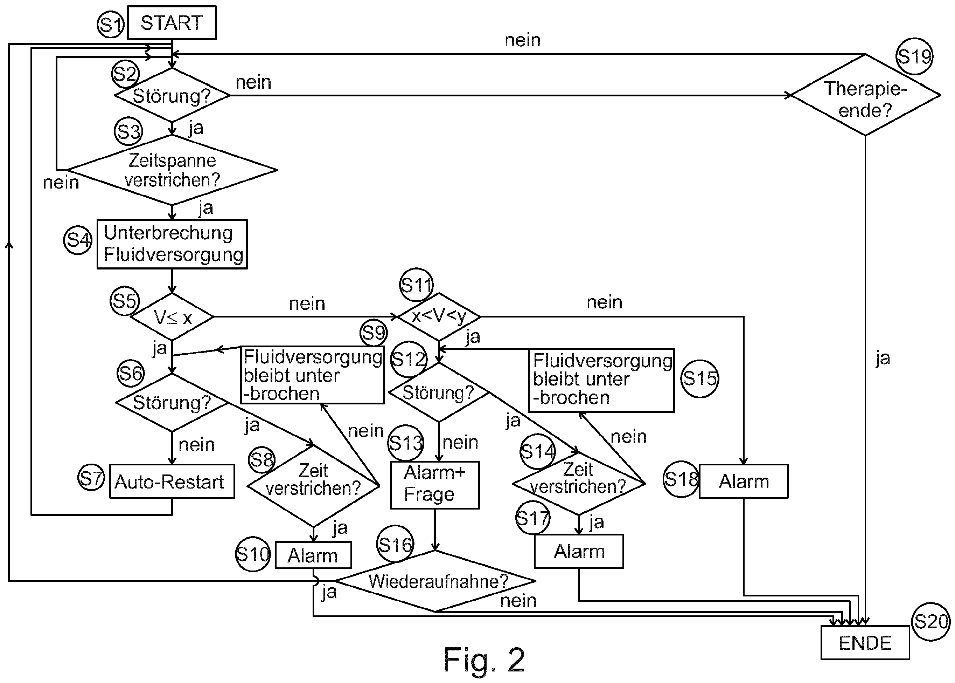

Fig. 2 zeigt den offenbarungsgemäßen Ablauf einer verbesserten Beutelgewichtüberwachung. Die Steuereinheit 68 der vorliegenden Offenbarung erhält grundsätzlich Informationen von einer Wägezelle (beispielsweise der ersten Wägezelle 62, der zweiten Wägezelle 64 und der dritten Wägezelle 66), welche das Gewicht eines Beutels (beispielsweise des Substitutionslösungsbeutels 34, des Sammelbeutels 40 und des Beutels 48), welcher eine Flüssigkeit/ ein Fluid enthält, kontinuierlich misst und überwacht. Aus diesen Informationen kann die Steuereinheit 68 bei "S2" feststellen, ob eine unerwartete, durch eine Störung hervorgerufenen Gewichtsvariation/ -schwankung/ -fluktuation/ -abweichung in dem Gewicht des Beutels 34, 40 oder 48 vorliegt.

Eine unerwartete, durch eine Störung hervorgerufene Gewichtsvariation wird von der Steuereinheit 68 in der Regel festgestellt, wenn von einer Wägezelle 62, 64 oder 66 eine abrupte Gewichtserhöhung oder Gewichtsverringerung festgestellt wird, welche größer oder gleich einem vorbestimmten Wert (beispielsweise 20 Gramm) ist.

Wenn eine Störung festgestellt wird ("ja"), überprüft die Steuereinheit 68 bei "S3", ob bereits eine vorbestimmte Zeitspanne (beispielsweise 10 Sekunden) seit erstmaligem Auftreten der Störung verstrichen ist. Wenn sich der Beutel 34, 40, 48 bzw. das Gewicht des Beutels 34, 40, 48 nicht innerhalb der vorbestimmten Zeitspanne stabilisiert, das heißt, wenn die Störung über die vorbestimmte Zeitspanne hinaus vorliegt, wird die Fluidversorgung durch Stoppen der Fluidpumpe 36, 46 oder 56 bei "S4" unterbrochen. Dies bedeutet, dass beispielsweise keine Flüssigkeit mehr aus dem Substitutionslösungsbeutel 34 durch die Substitutionslösungspumpe 36 herausgepumpt wird, dass beispielsweise keine Flüssigkeit mehr von der Ultrafiltratpumpe 46 in den Sammelbeutel 40 hineingepumpt wird, und dass beispielsweise keine Flüssigkeit mehr aus dem Beutel 48 durch die Pumpe 56 herausgepumpt wird.

Wenn sich das Gewicht des Beutels 34, 40 oder 48 hingegen bereits innerhalb der angesprochenen vorbestimmten Zeitspanne (insbesondere innerhalb von 10 Sekunden) stabilisiert (das heißt keine Störung mehr vorliegt, vgl. bei "S2"), erfolgt bevorzugt keine Unterbrechung der Fluidversorgung durch die Fluidpumpe 36, 46 oder 56.

Wenn die Fluidversorgung unterbrochen ist, wird bei "S5" überprüft, ob die Gewichtsvariation "V" einen vorbestimmten ersten Grenzwert x nicht überschreitet. Beispielsweise kann der vorbestimmte erste Grenzwert x auf 40 Gramm eingestellt sein. Wenn der vorbestimmte erste Grenzwert x nicht überschritten wird ("ja"), wird bei "S6" von der Steuereinheit 68 geprüft, ob die Störung verschwunden oder noch vorhanden ist. Solange die Störung vorliegt, wird die Unterbrechung der Fluidversorgung aufrechterhalten (siehe bei "S9"). Wenn sich der Beutel 34, 40 oder 48 bzw. das Gewicht des Beutels 34, 40 oder 48 innerhalb einer vorbestimmten Zeit (beispielsweise eine Minute) stabilisiert, das heißt keine Störung mehr vorliegt, wird die Fluidversorgung durch die Fluidpumpe 36, 46 oder 56 bei "S7" automatisch wieder bzw. neu gestartet. Stabilisiert sich der Beutel 34, 40 oder 48 nicht innerhalb der vorbestimmten Zeit, wird bei "S10" ein Alarm ausgegeben.

Wenn bei "S5" von der Steuereinheit 68 festgestellt wird ("nein"), dass die Gewichtsvariation "V" den vorbestimmten ersten Grenzwert x überschreitet, wird bei "S11" von der Steuereinheit 68 geprüft, ob die Gewichtsvariation "V" zwischen dem ersten vorbestimmten Grenzwert x und einem zweiten vorbestimmten Grenzwert y liegt. Der zweite vorbestimmte Grenzwert y kann beispielsweise auf 80 Gramm eingestellt werden. Liegt "V" in diesem Bereich ("ja"), wird bei "S12" von der Steuereinheit 68 geprüft, ob die Störung verschwunden oder noch vorhanden ist. Solange die Störung vorliegt, wird die Unterbrechung der Fluidversorgung aufrechterhalten (siehe bei "S15"). Wenn sich der Beutel 34, 40 oder 48 bzw. das Gewicht des Beutels 34, 40 oder 48 innerhalb einer vorbestimmten Zeit (beispielsweise eine Minute) stabilisiert, das heißt keine Störung mehr vorliegt, wird bei "S13" ein Alarm erzeugt und ein Anwender wird über die Benutzerschnittstelle 70 gefragt, ob er die Gewichtsvariation in die Fluidbilanz einberechnen möchte oder nicht. Optional kann gemäß der vorliegenden Offenbarung vorgesehen sein, dass sich der Anwender im Anschluss für eine Wiederaufnahme der Blutbehandlungstherapie entscheiden kann (bei "S16"). Stabilisiert sich der Beutel 34, 40 oder 48 nicht innerhalb der vorbestimmten Zeit, wird bei "S17" ein Alarm ausgegeben.

Wenn bei "S11" von der Steuereinheit 68 festgestellt wird, dass die Gewichtsvariation "V" über dem zweiten vorbestimmten Grenzwert y liegt ("nein" bei "S11"), wird bei "S18" ein Alarm ausgegeben.

Die vorliegende Routine endet, wenn der Alarm ausgegeben wird, oder wenn ein Therapieende erreicht wird, oder wenn sich der Anwender bei "S16" gegen eine Wiederaufnahme der Blutbehandlungstherapie entscheidet.

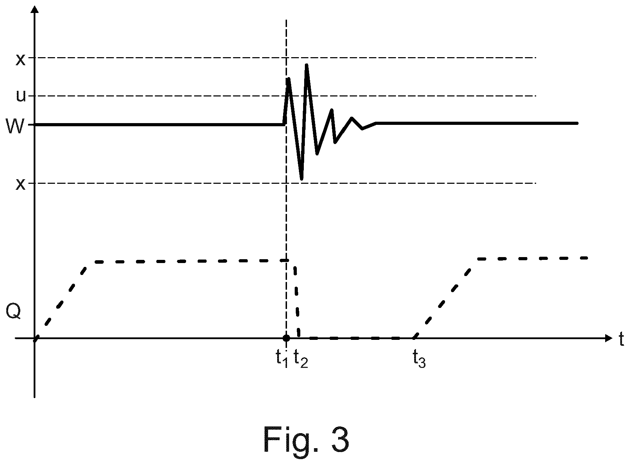

Fig. 3 zeigt ein Diagramm, in welchem ein zeitlicher Verlauf eines Beutelgewichts "W" und einer Fluidpumpen-Flussrate "Q", welche ein Fluid aus dem Beutel 34, 48 herauspumpt oder in den Beutel 40 hineinpumpt, gemäß der vorliegenden Offenbarung dargestellt. Zu Zeitpunkt t1 wird in Fig. 3 festgestellt, dass eine Gewichtsvariation des Beutelgewichts "W" großer einem vorbestimmten Wert u (beispielsweise 20 Gramm) ist. Die Steuereinheit 68 stellt somit zu Zeitpunkt t1 eine unerwartete Gewichtsvariation in einem Beutel 34, 40, 48 fest, da von einer Wägezelle 62, 64, 66 eine abrupte Gewichtserhöhung (größer dem vorbestimmten Wert u) erfasst wurde.

Wie aus Fig. 3 hervorgeht, wird die Flussrate Q einer Fluidpumpe 36, 46, 56 zu Zeitpunkt t1 noch nicht unmittelbar unterbrochen, sondern die Steuereinheit wartet noch eine vorbestimmte kurze Zeitspanne, beispielsweise etwa 10 Sekunden. Vorliegend stabilisiert sich das Beutelgewicht "W" nicht innerhalb der vorbestimmten kurzen Zeitspanne. Denn die Steuereinheit 68 stellt zu Zeitpunkt t2 fest, dass sich das Beutelgewicht "W" innerhalb der Zeitspanne nicht stabilisiert hat. Vor diesem Hintergrund stoppt die Steuereinheit 68 zu Zeitpunkt t2 die Fluidpumpe 36, 46, 56 und unterbricht somit die Fluidversorgung. Entsprechend verringert sich die Fluidpumpen-Flussrate "Q" zu Zeitpunkt t2 auf null.

Vorliegend stellt die Steuereinheit 68 in der Folge fest, dass der vorbestimmte erste Grenzwert x (beispielsweise 40 Gramm) der Gewichtsvariation nicht überschritten wird. Weiterhin stellt die Steuereinheit 68 fest, dass sich das Gewicht des Beutels 34, 40, 48 innerhalb der vorbestimmten Zeit (beispielsweise eine Minute) bis t3 wieder stabilisiert hat.

Da beide Bedingungen der vorliegenden Offenbarung erfüllt sind, also da sowohl die Gewichtsvariation den ersten vorbestimmten Grenzwert x nicht überschreitet als auch sich das Beutelgewicht "W" innerhalb der vorbestimmten Zeit stabilisiert, wird die Fluidversorgung durch die Fluidpumpe 36, 46, 56 automatisch wieder bzw. neu gestartet.

### Bezugszeichenliste

- 2: Blutbehandlungsvorrichtung/ Dialysemaschine
- 4: extrakorporaler Kreislauf
- 6: Dialysator
- 8: Dialysierflüssigkeitskreislauf
- 10: Membran
- 12: arterieller Abschnitt
- 14: venöser Abschnitt
- 16: arterielles Ende
- 18: arterieller Drucksensor
- 20: (arterielle) Blutpumpe
- 22: Dialysatoreingangsdrucksensor
- 24: venöses Ende
- 26: venöse Expansionskammer/ Luftfalle
- 28: Sicherheitsluftdetektor
- 30: Sicherheitsventil
- 32: venöser Drucksensor
- 34: Substitutionslösungsbeutel
- 36: Substitutionslösungspumpe
- 38: Abfluss
- 40: Sammelbeutel
- 42: Ultrafiltratdrucksensor
- 44: Blutleckdetektor
- 46: Ultrafiltratpumpe
- 48: Beutel
- 50: erstes Ventil
- 52: zweites Ventil
- 54: drittes Ventil
- 56: Pumpe
- 58: Flüssigkeitswärmer
- 60: Drucksensor
- 62: erste Wägezelle
- 64: zweite Wägezelle
- 66: dritte Wägezelle
- 68: Steuereinheit
- 70: Benutzerschnittstelle

## Patentansprüche

1. Blutbehandlungsvorrichtung (2) zur Verwendung in Blutbehandlungstherapien mit:
einem extrakorporalen Blutkreislauf (4), einem Dialysator (6) und einem Dialysierflüssigkeitskreislauf (8), wobei der extrakorporale Blutkreislauf (4) und der Dialysierflüssigkeitskreislauf (8) über eine in dem Dialysator (6) vorgesehene Membran (10), über welche Blut gefiltert werden kann, voneinander getrennt sind;
zumindest einem Beutel (34, 40, 48), welcher ein Fluid enthält;
zumindest einer Wägevorrichtung (62, 64, 66), welche eingerichtet ist, das Gewicht des zumindest einen Beutels (34, 40, 48) zu messen und zu überwachen; und
zumindest einer Fluidpumpe (36, 46, 56), welche eingerichtet ist, das Fluid aus dem Beutel (34, 40, 48) herauszupumpen oder in den Beutel (34, 40, 48) hineinzupumpen;
**gekennzeichnet durch**
eine Steuereinheit (68), welche eingerichtet ist:
eine Unterbrechung einer Fluidversorgung durch Stoppen der Fluidpumpe (36, 46, 56) zu bewirken, wenn eine unerwartete, durch eine Störung hervorgerufene und durch die Wägevorrichtung (62, 64, 66) gemessene Gewichtsvariation des Beutels (34, 40, 48) auftritt;
die Unterbrechung der Fluidversorgung temporär aufrechtzuerhalten, und zwar zumindest so lange, bis die Störung verschwindet;
die Fluidversorgung durch die Fluidpumpe (36, 46, 56) automatisch wieder bzw. neu zu starten, falls sich der Beutel (34, 40, 48) bzw. das Gewicht des Beutels (34, 40, 48) innerhalb einer vorbestimmten Zeit stabilisiert und die Gewichtsvariation einen ersten vorbestimmten Grenzwert nicht überschreitet; und
einen Alarm nur zu erzeugen, falls sich der Beutel (34, 40, 48) bzw. das Gewicht des Beutels (34, 40, 48) innerhalb der vorbestimmten Zeit nicht stabilisiert und/ oder die Gewichtsvariation den vorbestimmten ersten Grenzwert überschreitet.

2. Blutbehandlungsvorrichtung (2) nach Anspruch 1, **dadurch gekennzeichnet, dass** die Steuereinheit (68) eingerichtet ist, eine unerwartete Gewichtsvariation in dem Beutel (34, 40, 48) festzustellen, wenn von der Wägevorrichtung (62, 64, 66) eine abrupte Gewichtserhöhung oder Gewichtsverringerung festgestellt wird, wobei die Gewichtserhöhung oder Gewichtsverringerung größer oder gleich einem vorbestimmten Wert ist.

3. Blutbehandlungsvorrichtung (2) nach Anspruch 2, **dadurch gekennzeichnet, dass** der vorbestimmte Wert zwischen 20 Gramm und 35 Gramm eingestellt ist.

4. Blutbehandlungsvorrichtung (2) nach einem der vorhergehenden Ansprüche, **dadurch gekennzeichnet, dass** die Steuereinheit (68) eingerichtet ist, die Fluidversorgung durch Stoppen der Fluidpumpe (36, 46, 56) zu unterbrechen, wenn sich der Beutel (34, 40, 48) bzw. das Gewicht des Beutels (34, 40, 48) nicht innerhalb von einer vorbestimmten Zeitspanne stabilisiert.

5. Blutbehandlungsvorrichtung (2) nach einem der vorhergehenden Ansprüche, **dadurch gekennzeichnet, dass** die Steuereinheit (68) eingerichtet ist, falls die unerwartete Gewichtsvariation des Beutels (34, 40, 48) festgestellt bzw. erfasst wird, die Berechnung der Fluidbilanz sofort bzw. unmittelbar zu stoppen.

6. Blutbehandlungsvorrichtung (2) nach einem der vorhergehenden Ansprüche, **dadurch gekennzeichnet, dass** die Steuereinheit (68) eingerichtet ist, die Gewichtsvariation in die Fluidbilanz einzuberechnen, falls sich der Beutel (34, 40, 48) bzw. das Gewicht des Beutels (34, 40, 48) innerhalb der vorbestimmten Zeit stabilisiert und die Gewichtsvariation den ersten vorbestimmten Grenzwert nicht überschreitet.

7. Blutbehandlungsvorrichtung (2) nach einem der vorhergehenden Ansprüche, **dadurch gekennzeichnet, dass** die Steuereinheit (68) eingerichtet ist, falls die Gewichtsvariation den vorbestimmten ersten Grenzwert nicht überschreitet, das heißt kleiner oder gleich dem ersten vorbestimmten Grenzwert ist, und sich der Beutel (34, 40, 48) bzw. das Gewicht des Beutels (34, 40, 48) innerhalb der vorbestimmten Zeit stabilisiert, die Fluidversorgung durch die Fluidpumpe (36, 46, 56) automatisch wieder bzw. neu zu starten und die Gewichtsvariation in die Fluidbilanz einzuberechnen.

8. Blutbehandlungsvorrichtung (2) nach einem der vorhergehenden Ansprüche, **dadurch gekennzeichnet, dass** die Steuereinheit (68) eingerichtet ist, falls die Gewichtsvariation den vorbestimmten ersten Grenzwert überschreitet und einen vorbestimmten zweiten Grenzwert nicht überschreitet, das heißt zwischen dem ersten vorbestimmten Grenzwert und dem zweiten vorbestimmten Grenzwert liegt, und sich der Beutel (34, 40, 48) bzw. das Gewicht des Beutels (34, 40, 48) innerhalb der vorbestimmten Zeit stabilisiert, den Alarm zu erzeugen und einen Anwender über eine Benutzerschnittstelle (70) zu fragen, ob er die Gewichtsvariation in die Fluidbilanz einberechnen möchte oder nicht.

9. Blutbehandlungsvorrichtung (2) nach einem der vorhergehenden Ansprüche, **dadurch gekennzeichnet, dass** die Steuereinheit (68) eingerichtet ist, falls die Gewichtsvariation den vorbestimmten ersten Grenzwert überschreitet und einen vorbestimmten zweiten Grenzwert nicht überschreitet, das heißt zwischen dem ersten vorbestimmten Grenzwert und dem zweiten vorbestimmten Grenzwert liegt, und sich der Beutel (34, 40, 48) bzw. das Gewicht des Beutels (34, 40, 48) innerhalb der vorbestimmten Zeit nicht stabilisiert, den Alarm zu erzeugen, die Fluidversorgung unterbrochen zu halten und die Gewichtsvariation nicht in die Fluidbilanz einzuberechnen.

10. Blutbehandlungsvorrichtung (2) nach einem der vorhergehenden Ansprüche, **dadurch gekennzeichnet, dass** die Steuereinheit (68) eingerichtet ist, falls die Gewichtsvariation einen/ den zweiten vorbestimmten Grenzwert überschreitet, das heißt größer dem zweiten vorbestimmten Grenzwert ist, den Alarm zu erzeugen, die Fluidversorgung unterbrochen zu halten und die Gewichtsvariation nicht in die Fluidbilanz einzuberechnen.

## Claims

1. Blood treatment device (2) for use in blood treatment therapies, comprising:
an extracorporeal blood circuit (4), a dialyzer (6) and a dialysis fluid circuit (8), wherein the extracorporeal blood circuit (4) and the dialysis fluid circuit (8) are separated from each other via a membrane (10) provided in the dialyzer (6), via which blood can be filtered;
at least one bag (34, 40, 48), which contains a fluid;
at least one weighing device (62, 64, 66) configured to measure and monitor the weight of the at least one bag (34, 40, 48); and
at least one fluid pump (36, 46, 56) configured to pump the fluid out of the bag (34, 40, 48) or into the bag (34, 40, 48); and
**characterized by**
a control unit (68), which is configured:
to effect an interruption of a fluid supply by stopping the fluid pump (36, 46, 56) when an unexpected weight variation of the bag (34, 40, 48) caused by a malfunction and measured by the weighing device (62, 64, 66) occurs;
to temporarily maintain the interruption of the fluid supply, at least until the malfunction disappears;
to automatically restart or re-initiate the fluid supply by the fluid pump (36, 46, 56) if the bag (34, 40, 48) or the weight of the bag (34, 40, 48) stabilizes within a predetermined time and the weight variation does not exceed a first predetermined limit value; and
to generate an alarm only if the bag (34, 40, 48) or the weight of the bag (34, 40, 48) does not stabilize within the predetermined time and/or if the weight variation exceeds the predetermined first limit value.

2. Blood treatment device (2) according to claim 1, **characterized in that** the control unit (68) is configured to detect an unexpected weight variation in the bag (34, 40, 48) when an abrupt weight increase or weight decrease is detected by the weighing device (62, 64, 66), wherein the weight increase or weight decrease is equal to or greater than a predetermined value.

3. Blood treatment device (2) according to claim 2, **characterized in that** the predetermined value is set between 20 grams and 35 grams.

4. Blood treatment device (2) according to one of the preceding claims, **characterized in that** the control unit (68) is configured to interrupt the fluid supply by stopping the fluid pump (36, 46, 56) if the bag (34, 40, 48) or the weight of the bag (34, 40, 48) does not stabilize within a predetermined time period.

5. Blood treatment device (2) according to one of the preceding claims, **characterized in that** the control unit (68) is configured to stop the calculation of the fluid balance immediately or directly if the unexpected weight variation of the bag (34, 40, 48) is determined or detected.

6. Blood treatment device (2) according to one of the preceding claims, **characterized in that** the control unit (68) is configured to include the weight variation in the fluid balance if the bag (34, 40, 48) or the weight of the bag (34, 40, 48) stabilizes within the predetermined time and the weight variation does not exceed the first predetermined limit value.

7. Blood treatment device (2) according to one of the preceding claims, **characterized in that** the control unit (68) is configured to automatically restart or re-initiate the fluid supply by the fluid pump (36, 46, 56) and to calculate the weight variation into the fluid balance if the weight variation does not exceed the predetermined first limit value, i.e. is less than or equal to the first predetermined limit value, and the bag (34, 40, 48) or the weight of the bag (34, 40, 48) stabilizes within the predetermined time.

8. Blood treatment device (2) according to one of the preceding claims, **characterized in that** the control unit (68) is configured to generate the alarm and to ask a user via a user interface whether or not the weight variation should be included in the fluid balance if the weight variation exceeds the predetermined first limit value and does not exceed a predetermined second limit value, i.e. lies between the first predetermined limit value and the second predetermined limit value, and the bag (34, 40, 48) or the weight of the bag (34, 40, 48) stabilizes within the predetermined time.

9. Blood treatment device (2) according to one of the preceding claims, **characterized in that** the control unit (68) is configured to generate the alarm, to keep the fluid supply interrupted and not to include the weight variation in the fluid balance if the weight variation exceeds the predetermined first limit value and does not exceed a predetermined second limit value, i.e. lies between the first predetermined limit value and the second predetermined limit value, and the bag (34, 40, 48) or the weight of the bag (34, 40, 48) does not stabilize within the predetermined time.

10. Blood treatment device (2) according to one of the preceding claims, **characterized in that** the control unit (68) is configured to generate the alarm, to keep the fluid supply interrupted and not to include the weight variation in the fluid balance if the weight variation exceeds a/the second predetermined limit value, i.e. is greater than the second predetermined limit value.

## Revendications

1. Dispositif de traitement du sang (2) destiné à être utilisé dans des thérapies de traitement du sang avec :
une circulation sanguine extracorporelle (4), un dialyseur (6) et un circuit de liquide de dialyse (8), dans lequel la circulation sanguine extracorporelle (4) et le circuit de liquide de dialyse (8) sont séparés l'un de l'autre par l'intermédiaire d'une membrane (10) prévue dans le dialyseur (6), par l'intermédiaire de laquelle le sang peut être filtré ;
au moins un sac (34, 40, 48), lequel contient un fluide ;
au moins un dispositif de pesée (62, 64, 66), lequel est conçu pour mesurer et pour surveiller le poids du au moins un sac (34, 40, 48) ; et
au moins une pompe à fluide (36, 46, 56), laquelle est conçue pour pomper le fluide à l'extérieur du sac (34, 40, 48) ou le pomper à l'intérieur du sac (34, 40, 48) ;
**caractérisé par**
une unité de commande (68), laquelle est conçue :
pour provoquer une interruption d'une alimentation en fluide par arrêt de la pompe à fluide (36, 46, 56), lorsqu'une variation de poids inattendue du sac (34, 40, 48), provoquée par une perturbation et mesurée par le dispositif de pesée (62, 64, 66) se produit ;
pour maintenir temporairement l'interruption de l'alimentation en fluide, et ce au moins aussi longtemps que la perturbation n'a pas disparu ;
pour relancer ou redémarrer automatiquement l'alimentation en fluide par la pompe à fluide (36, 46, 56) si le sac (34, 40, 48) ou le poids du sac (34, 40, 48) se stabilise pendant une durée prédéfinie et la variation de poids ne dépasse pas une première valeur limite prédéfinie ; et
pour produire une alarme uniquement si le sac (34, 40, 48) ou le poids du sac (34, 40, 48) ne se stabilise pas pendant la durée prédéfinie et/ou la variation de poids dépasse la première valeur limite prédéfinie.

2. Dispositif de traitement du sang (2) selon la revendication 1, **caractérisé en ce que** l'unité de commande (68) est conçue pour déterminer une variation de poids inattendue dans le sac (34, 40, 48) lorsqu'une brusque augmentation de poids ou diminution de poids est déterminée par le dispositif de pesée (62, 64, 66), dans lequel l'augmentation de poids ou la diminution de poids est supérieure ou égale à une valeur prédéfinie.

3. Dispositif de traitement du sang (2) selon la revendication 2, **caractérisé en ce que** la valeur prédéfinie est réglée de manière comprise entre 20 grammes et 35 grammes.

4. Dispositif de traitement du sang (2) selon l'une quelconque des revendications précédentes, **caractérisé en ce que** l'unité de commande (68) est conçue pour interrompre l'alimentation en fluide par arrêt de la pompe à fluide (36, 46, 56), lorsque le sac (34, 40, 48) ou le poids du sac (34, 40, 48) ne se stabilise pas pendant un laps de temps prédéfini.

5. Dispositif de traitement du sang (2) selon l'une quelconque des revendications précédentes, **caractérisé en ce que** l'unité de commande (68) est conçue pour arrêter tout de suite ou immédiatement le calcul du bilan fluidique si la variation de poids inattendue du sac (34, 40, 48) est déterminée ou détectée.

6. Dispositif de traitement du sang (2) selon l'une quelconque des revendications précédentes, **caractérisé en ce que** l'unité de commande (68) est conçue pour prendre en compte la variation de poids dans le bilan fluidique si le sac (34, 40, 48) ou le poids du sac (34, 40, 48) se stabilise pendant la durée prédéfinie et la variation de poids ne dépasse pas la première valeur limite prédéfinie.

7. Dispositif de traitement du sang (2) selon l'une quelconque des revendications précédentes, **caractérisé en ce que** l'unité de commande (68) est conçue pour relancer ou redémarrer automatiquement l'alimentation en fluide par la pompe à fluide (36, 46, 56) et pour prendre en compte la variation de poids dans le bilan fluidique si la variation de poids ne dépasse pas la première valeur limite prédéfinie, c'est-à-dire est inférieure ou égale à la première valeur limite prédéfinie, et le sac (34, 40, 48) ou le poids du sac (34, 40, 48) se stabilise pendant la durée prédéfinie.

8. Dispositif de traitement du sang (2) selon l'une quelconque des revendications précédentes, **caractérisé en ce que** l'unité de commande (68) est conçue pour produire l'alarme et pour demander à un utilisateur par l'intermédiaire d'une interface utilisateur (70) s'il souhaiterait ou non prendre en compte la variation de poids dans le bilan fluidique si la variation de poids dépasse la première valeur limite prédéfinie et ne dépasse pas une deuxième valeur limite prédéfinie, c'est-à-dire se situe entre la première valeur limite prédéfinie et la deuxième valeur limite prédéfinie, et le sac (34, 40, 48) ou le poids du sac (34, 40, 48) se stabilise pendant la durée prédéfinie.

9. Dispositif de traitement du sang (2) selon l'une quelconque des revendications précédentes, **caractérisé en ce que** l'unité de commande (68) est conçue pour produire l'alarme, pour maintenir l'alimentation en fluide interrompue et pour ne pas prendre en compte la variation de poids dans le bilan fluidique si la variation de poids dépasse la première valeur limite prédéfinie et ne dépasse pas une deuxième valeur limite prédéfinie, c'est-à-dire est comprise entre la première valeur limite prédéfinie et la deuxième valeur limite prédéfinie, et le sac (34, 40, 48) ou le poids du sac (34, 40, 48) ne se stabilise pas pendant la durée prédéfinie.

10. Dispositif de traitement du sang (2) selon l'une quelconque des revendications précédentes, **caractérisé en ce que** l'unité de commande (68) est conçue pour produire l'alarme, pour maintenir l'alimentation en fluide interrompue et pour ne pas prendre en compte la variation de poids dans le bilan fluidique si la variation de poids dépasse une/la deuxième valeur limite prédéfinie, c'est-à-dire est supérieure à la deuxième valeur limite prédéfinie.
